# EUROPEAN PATENT APPLICATION

(11) **EP 4 848 298 A1**
(43) Date of publication of application: **02.12.2026**
(21) Application number: 25179096.0
(22) Date of filing: 27.05.2025
(51) Int. Cl.: G16H 20/40, G16H 50/20, G16H 50/50

(54) **SYSTEM AND METHOD FOR ELECTROMAGNETIC FIELD SIMULATION FOR MEDICAL DEVICES**

(71) Applicant: Siemens Healthineers AG, 91301 Forchheim (DE)
(72) Inventor: Audigier, Chloé, 3072 Ostermundigen (CH); Frings, Oliver, 91058 Erlangen (DE); Lluch, Eric, 90419 Nürnberg (DE); Meister, Felix, 91054 Erlangen (DE); Weiss, Maximilian, 90427 Nürnberg (DE)
(74) Representative: Siemens Healthineers Patent Attorneys

(57) **Abstract**

The present disclosure provides a computer-implemented method for electromagnetic field simulation. The method includes receiving input data representing a medical device geometry of a medical device, at least one operating parameter of the medical device, and an operating environment of the medical device; processing the input data using a trained neural operator model to determine a simulated electromagnetic field as generated by the medical device with the at least one operating parameter and in the operating environment; and calculating, based on the simulated electromagnetic field, at least one functional parameter of the medical device The method enables real-time simulation and optimization of electromagnetic fields for medical devices, particularly ablation devices, facilitating treatment planning and intra-procedural guidance.

## Description

### Technical field of the invention

The present disclosure relates to electromagnetic field simulation for medical devices, and more particularly to a system and method for fast electromagnetic field simulation using non-autoregressive neural operator models to optimize ablation device design and treatment planning.

### Background of the invention

Electromagnetic field simulation plays a crucial role in the development and optimization of medical devices, particularly in the field of ablation therapy. Ablation procedures utilize various energy modalities, such as radiofrequency or microwave, to treat tumors and other tissue abnormalities. These procedures rely on precise control of the electromagnetic field generated by the ablation device to achieve therapeutic outcomes while minimizing damage to surrounding healthy tissues.

Conventional approaches to electromagnetic field simulation for medical devices often involve complex numerical methods, such as finite element analysis or finite-difference time-domain techniques. These methods provide accurate results but can be computationally intensive, requiring significant time and resources to generate simulations for different device configurations and operating conditions. This computational burden can limit the ability to rapidly iterate on device designs or provide real-time guidance during medical procedures.

Additionally, the interaction between electromagnetic fields and biological tissues is complex, involving multiple physical phenomena such as heat transfer, tissue perfusion, and cellular response to thermal stress. Accurately modeling these interactions requires sophisticated Multiphysics simulations that further increase computational complexity and time requirements.

Another challenge in electromagnetic field simulation for medical devices is the variability in tissue properties across patients and anatomical locations (independent of the grammatical term usage, individuals with male, female or other gender identities are included within the term). Factors such as tissue composition, hydration status, and disease state can significantly affect the propagation and absorption of electromagnetic energy. Accounting for this variability in simulations adds another layer of complexity to the modeling process.

Furthermore, the optimization of ablation device designs and treatment protocols often requires numerous iterations of simulations with varying parameters. The time-consuming nature of conventional simulation methods can make it impractical to explore a wide range of design options or to tailor treatment plans to individual patient characteristics in a clinically relevant timeframe.

### Object of the invention

It has been appreciated that a system is needed that overcomes one or more of these problems.

### Solution according to the invention

In a first aspect, a computer-implemented method for electromagnetic field simulation is provided. The method comprises: receiving input data representing a medical device geometry of a medical device, at least one operating parameter of the medical device, and an operating environment of the medical device; processing the input data using a trained neural operator model to determine a simulated electromagnetic field as generated by the medical device with the at least one operating parameter and in the operating environment; and calculating, based on the simulated electromagnetic field, at least one functional parameter of the medical device.

As used herein, the term "medical device geometry" may refer to the three-dimensional structural configuration and physical characteristics of a medical device. This may include the device's shape, dimensions, material composition, and spatial arrangement of components. For example, in the context of an ablation device, the geometry may encompass the length and diameter of the device shaft, the shape and size of the electrode or antenna tip, and the configuration of any internal cooling channels or sensor placements. The medical device geometry may be represented in various formats, such as computer-aided design (CAD) models, voxelized representations, or finite element meshes, which can be used as inputs for electromagnetic field simulations.

As used herein, the at least one operating parameter of the medical device and the operating environment may together be referred to as the operating conditions of the medical device. The operating environment may refer to the geometry of the area of the patient where the device is to be applied (also referred to as the "3D material parameters"), while the operating parameters may refer to the variable parameters used to operate the medical device. The 3D material parameters may include a 3D image of the organ to be operated on, specific tissue properties such as electrical conductivity, thermal conductivity, and perfusion rates, as well as the spatial arrangement of different tissue types and structures surrounding the target area. The 3D material parameters may be defined in any suitable form, on a grid, as a point cloud, as a finite element mesh, as a voxelized representation, as a tetrahedral mesh, as a boundary element model, as a structured or unstructured mesh, or as a combination of different representation formats depending on the specific tissue regions being modeled. Some of these parameters may vary spatially and temporally during a procedure. The variable parameters used to operate the medical device may include power level, frequency, duration of application, pulse width, duty cycle, cooling flow rate, electrode configuration, and treatment mode selection. The operating environment may also encompass environmental factors such as ambient temperature or the presence of cooling fluids. These parameters collectively form the environment input in the system architecture and serve as inputs to the neural operator model. The variable parameters and environmental factors directly influence the electromagnetic field generated by the device and consequently affect the resulting ablation zone characteristics in the case of an ablation device. In some implementations, the operating conditions may be dynamically adjusted in real-time based on feedback from the simulation process, allowing for adaptive treatment strategies.

As used herein, the at least one functional parameter of the medical device may be a characteristic of the simulated electromagnetic field (e.g. a power density distribution). It may be a calculated parameter such as a radius or other characteristic dimension of an ablation zone or an amount or density distribution of energy that is released into tissue, in case of an ablation device. It may be an overlap between the shape of the simulated electromagnetic field or the ablation zone and a target structure in the operating environment such as a tumor to be treated. Generally, a functional parameter of the medical device may be an output characteristic of the medical device that is useful for one of device design optimization, treatment planning, real-time treatment parameter adjustment, and tissue-specific optimization. This is not an exhaustive list, the skilled person will be able to determine further useful functional parameters of the simulated medical device that can be calculated based on the simulated electromagnetic field and the further input data.

This method enables rapid and accurate simulation of electromagnetic fields generated by medical devices, facilitating efficient device design optimization and treatment planning without requiring time-consuming conventional numerical simulations or extensive physical prototyping and testing.

The medical device may be an ablation device, and the at least one functional parameter may comprise a parameter of an ablation zone of the ablation device and/or a power density within the ablation zone and/or a parameter of the simulated electromagnetic field. By simulating ablation zones and power densities, the method provides critical information for optimizing treatment efficacy and safety in medical procedures involving electromagnetic ablation.

The trained neural operator model may be a non-autoregressive neural operator model. A non-autoregressive neural operator model comprises a deep learning architecture engineered to approximate mappings between infinite-dimensional function spaces, thereby facilitating simultaneous prediction of solutions across entire spatial or temporal domains without sequential generation. The architecture employs parallel computation structures that efficiently solve partial differential equations (PDEs), including Maxwell's equations, with superior computational efficiency compared to traditional numerical methods.

The non-autoregressive neural operator model may comprise: a branch network configured to process the input data, a trunk network configured to process space and time sampling locations, and an attention mechanism combining the branch network and the trunk network. This architecture enables efficient processing of complex spatial and temporal data, allowing for accurate simulation of electromagnetic fields across diverse device geometries and operating conditions.

The branch network processes input functions, such as boundary conditions and material properties, through deep neural networks to extract latent features. Concurrently, the trunk network encodes spatial-temporal coordinates into a compatible latent space, establishing basis functions for solution representation. These components operate jointly to capture the underlying physics of the problem domain. The model performs its core operation by combining the branch and trunk network outputs using an attention mechanism such as, typically, a dot product.

When applied specifically to Maxwell's equations, the branch network processes electromagnetic field boundary conditions, while the trunk network evaluates solutions at arbitrary points within the computational domain. This architectural configuration circumvents iterative solving procedures typically required by conventional numerical methods, thereby enabling real-time simulations of electromagnetic wave propagation in complex media.

The non-autoregressive nature of the model permits parallel evaluation at all query points, resulting in significant computational advantages over sequential approaches and allowing efficient use of graphics processing unit (GPU) clusters or high-performance computing (HPC) clusters. It is possible to omit calculations for query points that are not relevant to the medical application, making the model more efficient than conventional numerical methods that must be applied across the entire domain. It is also not necessary to compute for consecutive points in time, the model can be applied to any point in time. An additional advantage is that the model achieves mesh-independence through coordinate encoding, allowing for flexible discretization schemes without requiring retraining.

The branch network may be configured to process input data in at least one format selected from a 3D grid, a finite element mesh, and a point cloud. This flexibility in input data formats allows the method to accommodate various representations of medical device geometries and tissue properties, enhancing its applicability across different simulation scenarios.

The trained neural operator model may have been trained using a combination of supervised learning with reference electromagnetic field data and physics-informed learning based on Maxwell's equations. This hybrid training approach ensures that the model captures both empirical relationships from real-world data and fundamental physical principles, resulting in more accurate and generalizable simulations.

The method may further comprise optimizing the medical device geometry by: defining a desired electromagnetic field pattern; calculating a loss function based on a difference between the simulated electromagnetic field and the desired electromagnetic field pattern; and updating the input data representing the medical device geometry using a gradient-based optimization method to minimize the loss function. This optimization process enables automated refinement of medical device designs to achieve specific electromagnetic field characteristics, potentially improving treatment outcomes and device performance.

The medical device may be an ablation device, and the desired electromagnetic field pattern may correspond to a spherical ablation zone of the ablation device. Optimizing for a spherical ablation zone can simplify treatment planning and improve the predictability of ablation procedures, potentially enhancing therapeutic outcomes.

The method may be performed in real-time during a medical procedure to provide treatment guidance, further comprising: receiving updated input data reflecting at least one of an updated operating parameter of the medical device and an updated operating environment of the medical device; re-simulating the electromagnetic field based on the updated input data; and providing real-time feedback for adjusting treatment parameters.

Real-time simulation and feedback capabilities enable dynamic adaptation of treatment parameters during procedures, potentially improving treatment precision and safety.

The method may be performed as part of a pre-procedure treatment planning process, further comprising: simulating multiple electromagnetic fields for different medical device placements; evaluating the simulated electromagnetic fields to determine an optimal medical device placement; and generating a treatment protocol based on the optimal medical device placement.

This pre-procedure planning capability can help clinicians optimize device placement and treatment parameters before the actual procedure, potentially improving treatment efficacy and reducing procedural complications.

The method may further comprise optimizing device parameters for a specific tissue type by: simulating the electromagnetic field for multiple sets of at least one operating parameter of the medical device; evaluating the simulated electromagnetic field to determine an optimal set of the at least one operating parameter of the medical device for the specific tissue type; and storing the optimal set of the at least one operating parameter of the medical device for future use with the specific tissue type.

This tissue-specific optimization approach allows for personalized treatment strategies that account for variations in tissue properties, potentially enhancing treatment outcomes across diverse patient populations.

The method may further comprise integrating the simulated electromagnetic field with a biophysical model for heat propagation and cell necrosis to predict a resulting ablation zone.

By combining electromagnetic field simulation with biophysical models, the method provides a more comprehensive prediction of treatment effects, enabling more accurate planning and optimization of ablation procedures.

The method may further comprise optimizing the at least one operating parameter of the medical device to maximize overlap between a predicted ablation zone and a target tumor volume.

This optimization approach helps ensure that the ablation zone effectively covers the entire tumor while minimizing damage to surrounding healthy tissue, potentially improving treatment efficacy and reducing side effects.

In a second aspect, a computer device comprising a processor and a memory is provided. The memory comprises instructions stored thereon which, when executed by the processor, cause the computer device to perform the method of the first aspect.

This computer device implementation enables practical deployment of the electromagnetic field simulation method in clinical and research settings, providing a powerful tool for treatment planning and device optimization.

In a third aspect, a computer program comprising instructions is provided. When executed by a computer, the instructions cause the computer to carry out the method of the first aspect.

This computer program implementation allows for widespread distribution and utilization of the electromagnetic field simulation method across various computing platforms.

In a fourth aspect, a computer-readable medium comprising instructions is provided. When executed by a computer, the instructions cause the computer to carry out the method of the first aspect.

This computer-readable medium implementation facilitates easy distribution and installation of the electromagnetic field simulation method on diverse computing systems.

### Brief description of the drawings

Embodiments of the invention will be described, by way of example, with reference to the following drawings, in which:
FIG. 1a illustrates a block diagram of a system architecture for electromagnetic field simulation, according to an embodiment of the disclosure.
FIG. 1b illustrates a block diagram of a system architecture for electromagnetic field simulation and optimization, according to an embodiment of the disclosure.
FIG. 2 illustrates a simulation system comprising parallel processing paths, according to an embodiment of the disclosure.
FIG. 3 illustrates a perspective view of an ablation device and its associated ablation zone, according to an embodiment of the disclosure.
FIG. 4 illustrates a flowchart depicting a model training process for a neural operator network, according to an embodiment of the disclosure.
FIG. 5 illustrates a flowchart depicting a method for electromagnetic field simulation, according to an embodiment of the disclosure.
FIG. 6 illustrates a flowchart depicting a device design optimization method, according to an embodiment of the disclosure.
FIG. 7 illustrates a flowchart depicting a method for real-time treatment parameter adjustment, according to an embodiment of the disclosure.
FIG. 8a illustrates a flowchart depicting a pre-procedure planning workflow, according to an embodiment of the disclosure.
FIG. 8b illustrates a flowchart depicting an intra-procedure guidance workflow, according to an embodiment of the disclosure.
FIG. 8c illustrates a flowchart depicting a device design optimization workflow, according to an embodiment of the disclosure.
FIG. 8d illustrates a flowchart depicting a tissue-specific parameter optimization workflow, according to an embodiment of the disclosure.
FIG. 9 illustrates a system architecture for electromagnetic field simulation, according to an embodiment of the disclosure.

Common reference numerals are used throughout the figures to indicate similar features.

### Description of the preferred embodiments

The physics governing electromagnetic field simulation and thermal ablation involve complex interactions between electromagnetic waves, heat transfer, and biological tissue responses. Electromagnetic field simulation in the context of medical devices, particularly ablation devices, is based on Maxwell's equations. In the frequency domain, the governing equation for electromagnetic wave propagation in three dimensions is expressed as: $∇ \times \left({μ}_{r}^{- 1}∇\times E\right) - \left({ω}^{2}/{c}_{0}^{2}\right) \left({ε}_{r}-jσ/{ωε}_{0}\right) E = 0$ (1) where ${μ}_{r}^{- 1}$ is the relative permeability, *εᵣ* is the relative permittivity, *σ* is the electrical conductivity in S/m, *ω* is the angular frequency in rad/s, *c*₀ is the speed of light in vacuum, and E is the electric field strength in V/m.

For simulating the microwave source in ablation devices, S-parameter port boundary conditions may be applied: $S = \frac{{\int }_{port 1} \left(\left({E}_{e}-{E}_{1}\right){E}_{1}^{∗}\right) {dA}_{1}}{{\int }_{port 1} \left(\left({E}_{e}∗{E}_{1}\right){E}_{1}^{∗}\right) {dA}_{1}}$ (2) where *Eₑ* is the excitation plus the reflected field, *E*₁ is the electric field of the port, and *A*₁ is the area of the port boundary.

To predict the resulting ablation zone, the simulated electromagnetic field may be integrated with a biophysical model for heat propagation and cell necrosis. Heat transfer in biological tissues during thermal ablation is commonly modeled using Pennes' Bioheat equation: ${ρC}_{p} \frac{\partial T}{\partial t} = ∇ ⋅ k ∇ T + \left({ρ}_{b}{C}_{p , b}{ω}_{b}\right) \left(T-{T}_{b}\right) + \frac{1}{2} σ {\left|E\right|}^{2}$ (3) where *ρ* is mass density in kg/m³, *Cₚ* is the isobaric heat capacity in J/kg·K, *k* is thermal conductivity in W/m·K, *T* is temperature in K, and the subscript b denotes properties of blood. The term $\frac{1}{2} σ {\left|E\right|}^{2}$ represents heat generation due to absorbed electromagnetic energy in W/m³.

Boundary conditions for the bioheat equation may include a convective heat flux condition at internal boundaries between the ablation device and surrounding tissue: $\vec{n} ⋅ \left(-k∇T\right) = h ⋅ \left(T-{T}_{ext}\right)$ (4) where *̅n̅*̅ is the normal vector to the element, *h* is the heat transfer coefficient in W/m²·K, and *Tₑₓₜ* is the external temperature.

The degree of thermal tissue injury may be quantified using the Arrhenius damage integral: $α \left(t\right) = {α}_{0} + {\int }_{0}^{t} \left({Ae}^{- ΔE / RT \left(t′\right)}\right) dt ′$ (5) where *α*(*t*) is the degree of tissue injury over time, *α*₀ is initial injury, *A* is the frequency factor in 1/s, *ΔE* is the activation energy to induce tissue damage in J/mol, R is the universal gas constant in J/mol·K, and *T*(*t*) is the temperature history of the tissue in K.

The fraction of damaged tissue (*θ_{d}*) may be determined by: ${θ}_{d} = 1 - {e}^{- α \left(t\right)}$ (6) where *θ_{d}* represents the percentage of cell death.

By integrating the electromagnetic field simulation with these biophysical models for heat propagation and cell necrosis, a comprehensive prediction of the resulting ablation zone may be obtained. This integrated approach allows for accurate simulation of the complex physical processes involved in thermal ablation procedures.

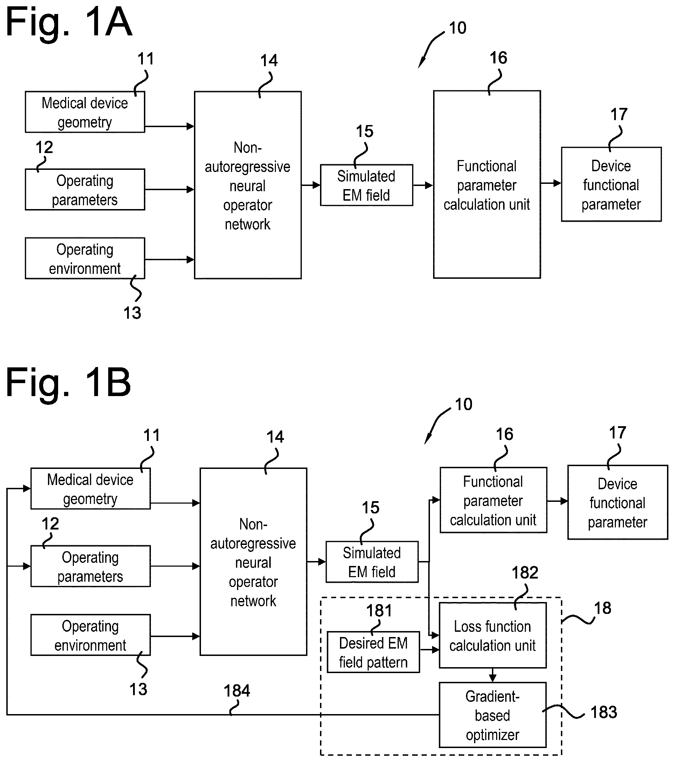

FIG. 1a illustrates a system architecture 10 for electromagnetic field simulation. The system architecture 10 includes a geometry input 11, a parameter input 12, and an environment input 13, which provide input data to a neural network module 14.

A geometry input 11 receives input data representing a three-dimensional geometric configuration of a medical device, such as an ablation device. Such input data will also be referred to as input data representing a medical device geometry. A parameter input 12 receives data defining operational settings of the medical device, which may include power levels, frequency, and duration. Such input will also be referred to as input data representing at least one operating parameter of the medical device. An environment input 13 receives data characterizing ambient conditions and material properties of the surrounding medium in which the medical device operates. Such input will also be referred to as input data representing an operating environment of the medical device. The inputs of parameter input 12 and material properties or environment input 13 together may be referred to as operating conditions, as defined earlier.

The neural network module 14 implements a trained neural operator model. The trained neural operator model may be a trained non-autoregressive neural operator model configured to process the input data from the geometry input 11, parameter input 12, and environment input 13. The neural network module 14 simulates an electromagnetic field generated by the medical device under the specified conditions as specified by the inputs.

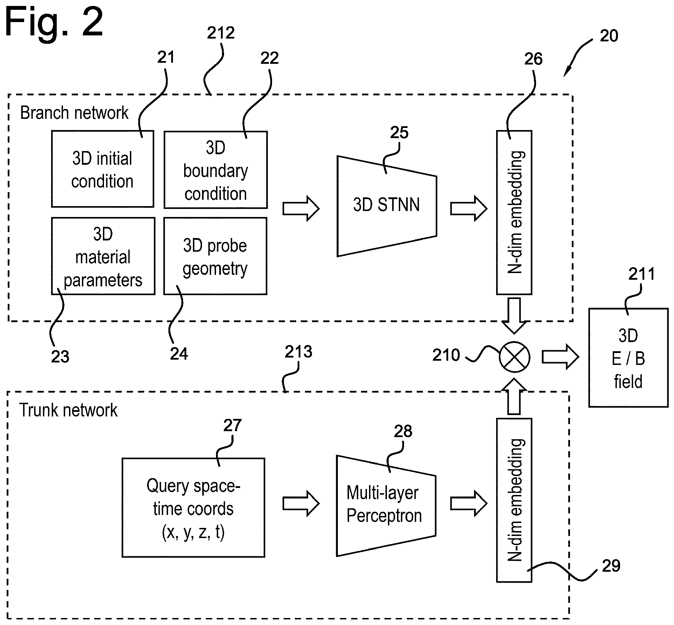

The neural network module 14 outputs a simulated electromagnetic (EM) field 15. As shown in Fig. 2, the simulated EM field is produced by combining outputs from a branch network 212 and trunk network 213 through an attention mechanism 210, such as a dot product operation. The field output 211 generates a three-dimensional electromagnetic field distribution representing the simulated field in the spatial domain surrounding the medical device.

The simulated electromagnetic field 15 is transmitted to a calculation module 16. The calculation module 16 processes the electromagnetic field data to derive relevant metrics and performance indicators. The specific metrics calculated will depend on the particular use case for which the simulation is being utilized, as will be discussed in more detail in the context of various use case examples.

A parameter output 17 receives the processed data from the calculation module 16. The parameter output 17 provides one or more functional parameters that characterize the performance of the medical device under the specified conditions. These functional parameters, depending on use case, may be one or more of characterizing parameters, such as dimensions, of the ablation zone, a power density distribution (e.g. within the ablation zone), a parameter of the simulated electromagnetic field, or other quantities, such as indicators of therapeutic efficacy and safety. The functional parameter can be a single number, a vector, a two-dimensional or 3-dimensional field of vectors, a tensor, or any other relevant representation.

The system architecture 10 enables simulation of electromagnetic fields and calculation of functional parameters based on the provided input data through the sequential processing of information through the connected components 11-17. This architecture allows for rapid and accurate prediction of electromagnetic field distributions and resulting functional parameters for medical devices, facilitating device design optimization and treatment planning, in ways that are not possible through traditional EM equation solving.

FIG. 1b illustrates an enhanced system architecture 10 for electromagnetic field simulation and optimization. The system architecture 10 builds upon the components described in FIG. 1a, with the addition of an optimization subsystem 18 that enables iterative refinement of device parameters.

The system architecture 10 receives input through the geometry input 11, the parameter input 12, and the environment input 13, as previously described. These inputs are processed by the neural network module 14 to generate a simulated EM field 15. The simulated EM field 15 is then provided to the calculation module 16, which produces the parameter output 17.

The optimization subsystem 18 comprises several interconnected components designed to refine the input parameters based on desired outcomes. A desired electromagnetic field pattern 181 defines target electromagnetic field specifications or desired ablation zone characteristics. For example, the desired electromagnetic field pattern 181 may specify a spherical ablation zone, which can simplify treatment planning and improve therapeutic outcomes.

A loss calculator 182 compares the simulated EM field 15 with the desired electromagnetic field pattern 181. The loss calculator 182 quantifies the discrepancy between the simulated field and the desired pattern, providing a measure of how closely the current device configuration achieves the target outcome.

An optimizer 183 processes the output from the loss calculator 182 to determine parameter adjustments. The optimizer 183 employs gradient-based optimization techniques to calculate updates to the input parameters that minimize the discrepancy between the simulated and desired field patterns.

A feedback path 184 connects the optimizer 183 back to the input components (geometry input 11, parameter input 12, and environment input 13). The feedback path 184 enables the system to iteratively refine the device geometry and operating parameters based on the optimization results.

The optimization process leverages the differentiability of the neural network module 14. Unlike traditional ordinary differential equation (ODE) and partial differential equation (PDE) solving systems, the neural network approach allows for automatic differentiation through the entire simulation pipeline. This differentiability enables the use of backpropagation, a technique where gradients of the loss function with respect to input parameters are computed efficiently by propagating error gradients backward through the network.

Backpropagation allows the system to calculate how small changes in input parameters affect the output electromagnetic field and resulting ablation zone. By iteratively adjusting these parameters in the direction that minimizes the loss function, the system can optimize the device geometry and operational settings to achieve desired outcomes, such as a spherical ablation zone or maximized overlap between the predicted ablation zone and a target tumor volume.

The enhanced system architecture 10 with the optimization subsystem 18 enables a closed-loop optimization process. The system can continuously simulate electromagnetic fields, evaluate outcomes, and refine input parameters to improve device performance or treatment efficacy. This capability allows for rapid design iterations and parameter tuning that may be challenging or time-consuming with traditional electromagnetic simulation approaches.

FIG. 2 illustrates a simulation system 20 comprising a branch network 212 and a trunk network 213 arranged in parallel processing paths. The simulation system 20 implements a non-autoregressive neural operator model for electromagnetic field simulation.

The branch network 212 is configured to process spatially varying input data. The branch network 212 includes multiple input components for processing three-dimensional data. These components include an initial condition input 21, a boundary condition input 22, material parameters input 23, and a probe geometry input 24. The initial condition input 21 receives and encodes initial electromagnetic field distributions. The boundary condition input 22 processes domain constraints including Dirichlet and Neumann boundary conditions. The material parameters input 23 encodes spatially varying material properties such as permittivity, permeability, and electrical conductivity of tissues. The probe geometry input 24 processes the geometric configuration of the ablation device or other electromagnetic field generator.

The outputs from the initial condition input 21, boundary condition input 22, material parameters input 23, and probe geometry input 24 are provided to a neural network processor 25. The neural network processor 25 comprises a 3D spatio-temporal neural network configured to process and integrate the multidimensional input data. The neural network processor 25 may be implemented using convolutional neural layers for grid-structured data, graph convolutional layers for mesh-based representations, or point-based networks for point cloud data, depending on the specific discretization of the input domain.

This flexibility allows the branch network 212 to process input data in formats including 3D grid, finite element mesh, or point cloud representations.

The processed output from the neural network processor 25 is transmitted to a branch embedding module 26. The branch embedding module 26 transforms the integrated spatio-temporal information into a fixed-length feature vector that encapsulates the essential characteristics of the input physical system.

The trunk network 213 operates in parallel with the branch network 212. The trunk network 213 is configured to process space and time sampling locations. The trunk network 213 begins with a coordinate input module 27 that receives query space-time coordinates. These coordinates specify points in space and time where electromagnetic field values are to be evaluated. The coordinates from the coordinate input module 27 are processed through a perceptron processor 28. The perceptron processor 28 comprises a multi-layer perceptron that transforms the coordinates through multiple dense layers with nonlinear activation functions. The output from the perceptron processor 28 is transmitted to a trunk embedding module 29. The trunk embedding module 29 produces another fixed-length feature vector encoding the query location information.

The outputs from the branch embedding module 26 and trunk embedding module 29 are combined through an attention mechanism 210, such as a dot product. The attention mechanism 210 operation implements the core of the operator learning framework, merging information about the physical system with specific query locations to predict field values. In an embodiment, the attention mechanism 210 also comprises an offset. The result of the attention mechanism 210 operation is provided to the field output 211, which produces the three-dimensional electromagnetic field distribution at the queried locations.

The non-autoregressive neural operator model implemented in the simulation system 20 may be based on the DeepONet architecture (Lu, L., Jin, P., Pang, G., Zhang, Z. & Karniadakis, G. E. "Learning nonlinear operators via DeepONet based on the universal approximation theorem of operators", Nature Machine Intelligence 3, 218-229 (2021)), which is designed to learn nonlinear operators efficiently. This architecture leverages the universal approximation theorem for operators, which states that a neural network with a single hidden layer can approximate any nonlinear continuous operator to arbitrary accuracy.

In the DeepONet framework, the branch network 212 and trunk network 213 work in tandem to process input functions and output locations separately. The branch network 212 may encode the input function at a fixed number of sensor points, while the trunk network 213 encodes the locations for the output function evaluation. This separation allows the model to handle varying input and output dimensions effectively.

The neural network processor 25 in the branch network 212 may be implemented using various architectures beyond the exemplary 3D spatio-temporal neural network (STNN). For instance, a Fourier neural operator (FNO) may be employed, which leverages the Fourier transform to capture global dependencies in the input data. Alternatively, a graph neural network (GNN) may be used to process input data represented as graphs, allowing for flexible handling of irregular geometries and non-uniform discretizations.

In place of the multi-layer perceptron in the perceptron processor 28 of the trunk network 213, other architectures may be utilized. For example, a radial basis function network (RBFN) could be employed to capture nonlinear relationships between spatial coordinates and field values. Another option may be to use a residual network (ResNet) architecture, which can facilitate the training of deeper networks and potentially improve the model's ability to learn complex coordinate mappings.

The attention mechanism 210 operation combining the outputs of the branch and trunk networks allows the model to selectively focus on relevant aspects of the input function when predicting field values at specific locations. In an embodiment, the attention mechanism 210 operation is the dot product. In some embodiments, alternative attention mechanisms may be used to enhance the model's ability to capture complex relationships between inputs and outputs, such as multi-head attention, scaled dot-product attention, additive attention, self-attention, and cross-attention mechanisms. These alternative attention mechanisms can provide different ways of weighting the importance of various input features when generating the electromagnetic field predictions, potentially improving accuracy for complex device geometries and tissue environments.

By leveraging these advanced neural network architectures and techniques, the simulation system 20 may achieve high accuracy in electromagnetic field prediction while maintaining computational efficiency. The flexibility of the DeepONet framework allows for adaptation to various types of input data and problem domains, making it suitable for a wide range of electromagnetic simulation tasks in medical device design and optimization.

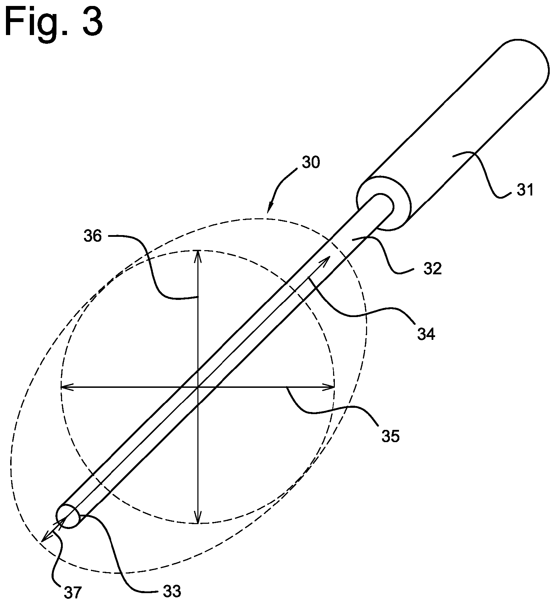

FIG. 3 illustrates a perspective view of an ablation device 31 and an associated ablation zone 30. The ablation device 31 comprises an elongated antenna shaft 32 that extends longitudinally and terminates at an antenna tip 33.

The ablation zone 30 is represented as a three-dimensional ellipsoidal volume surrounding the antenna shaft 32. The ablation zone 30 is characterized by several dimensional parameters. An ablation length 34 extends along the longitudinal axis of the antenna shaft 32. An ablation long width 35 represents the maximum diameter of the ablation zone 30 in one direction perpendicular to the antenna shaft 32. An ablation short width 36 indicates another diameter measurement of the ablation zone 30 in a direction perpendicular to both the antenna shaft 32 and the ablation long width 35.

A distal boundary 37 represents the distance that the ablation zone 30 extends beyond the antenna tip 33. This measurement indicates how far the ablation effect reaches past the physical end of the ablation device 31.

The dimensional parameters of the ablation length 34, ablation long width 35, and ablation short width 36 define the three-dimensional extent of the ellipsoidal ablation zone 30 generated by the ablation device 31. These parameters may be calculated by the calculation module 16 based on the simulated electromagnetic field 15 produced by the neural network module 14, as described in relation to FIG. 1a and FIG. 1b.

The simulated electromagnetic field generated by equation (1) may be used to determine the extent of the ablation zone 30, with higher precision than the assumption of a spherical field often used in prior art approaches. The electric field strength E calculated from this equation may be a key factor in determining the boundaries of the ablation length 34, ablation long width 35, and ablation short width 36.

The S-parameter port boundary conditions described by equation (2) may be applied to model the antenna tip 33 and how it radiates electromagnetic energy into the surrounding tissue, influencing the shape and size of the ablation zone 30.

Equation (3), representing Pennes' Bioheat equation, may be used to predict how the electromagnetic energy deposited in the tissue translates into temperature rise. The temperature distribution calculated from this equation may help define the boundaries of the ablation zone 30, as tissue necrosis occurs at specific temperature thresholds.

The convective heat flux boundary condition described by equation (4) may be applied at the interface between the antenna shaft 32 and the surrounding tissue, affecting how heat propagates from the device into the tissue and consequently influencing the shape of the ablation zone 30.

Equations (5) and (6), which quantify the degree of thermal tissue injury, may be used to determine or define the boundaries of the ablation zone 30. By calculating the fraction of damaged tissue θ_{d} at different points in space, the system may delineate where the ablation length 34, ablation long width 35, ablation short width 36, and distal boundary 37 should be placed.

The integration of these equations in the simulation process may allow for accurate prediction of the three-dimensional ellipsoidal volume of the ablation zone 30 and its relationship to the physical geometry of the ablation device 31. This integration may enable the system to provide quantitative estimates of the ablation zone dimensions and enables quantitative analysis of the predicted ablation effect. This analysis may be used in various applications, such as treatment planning, device optimization, and real-time procedure guidance, as facilitated by the system architecture 10 and simulation system 20 described in previous figures.

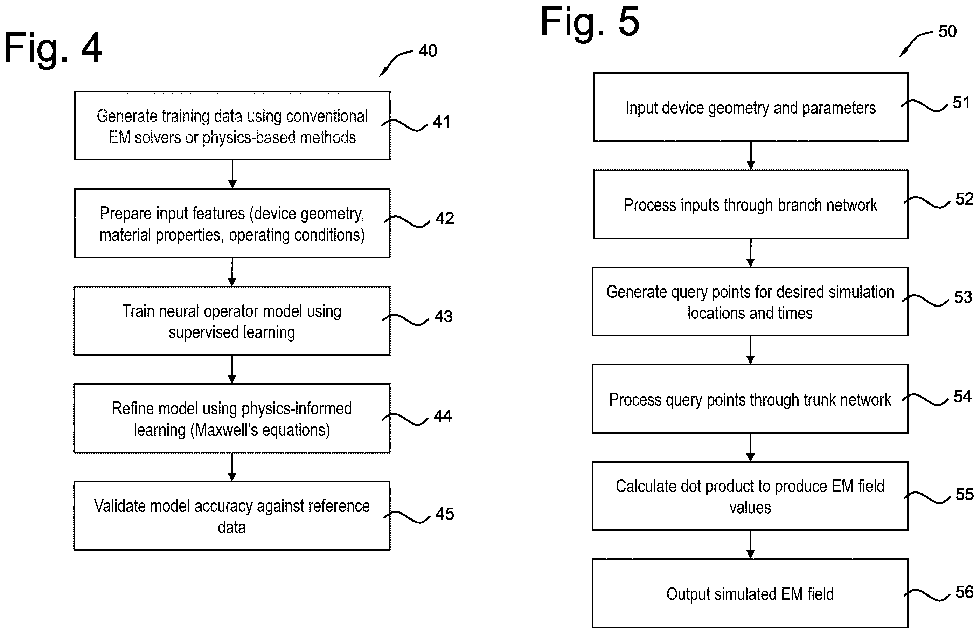

FIG. 4 illustrates a model training process 40 for developing a non-autoregressive neural operator model for electromagnetic field simulation. The model training process 40 comprises a sequence of steps designed to create an accurate and computationally efficient electromagnetic field simulator.

A training data generation step 41 produces a dataset of electromagnetic field solutions using conventional electromagnetic solvers or physics-based methods. These reference solutions may be generated using established numerical techniques such as finite element methods, finite difference time domain methods, or the method of moments. Commercial electromagnetic solvers may be employed to simulate Maxwell's equations for various ablation device configurations, producing high-fidelity electromagnetic field distributions that serve as ground truth for model training.

An input feature preparation step 42 processes and standardizes the input parameters that characterize each simulation scenario. These parameters include the three-dimensional geometric representation of the ablation device, which may be encoded as voxelized binary arrays, tetrahedral meshes, or point clouds depending on the specific implementation. The input features are typically normalized and structured to conform to the expected input format of the neural network module 14.

A supervised learning step 43 trains the neural operator model using the prepared input features and corresponding reference electromagnetic field solutions. The supervised training process involves minimizing a loss function that quantifies the discrepancy between the model predictions and reference solutions, typically using variants of stochastic gradient descent optimizers.

A physics-informed optional refinement step 44 enhances the model's accuracy and generalizability by incorporating direct knowledge of the governing physical laws. The physics-informed refinement step 44 integrates Maxwell's equations as additional constraints in the training objective. This step involves computing the residuals of Maxwell's equations at randomly sampled collocation points within the domain and including these residuals in the loss function, thereby penalizing solutions that violate the underlying physical principles.

An optional model validation step 45 evaluates the trained neural operator model against independent reference data not used during the training process. Validation metrics may include point-wise relative error in predicted field values, integrated error norms across the spatial domain, and specific performance indicators relevant to ablation procedures such as accuracy in predicted ablation zone dimensions or power density distributions.

The model training process 40 combines supervised learning with physics-informed refinement to develop a neural operator model capable of accurately simulating electromagnetic fields. The supervised learning step 43 allows the model to learn from high-fidelity reference data, while the physics-informed refinement step 44 ensures consistency with fundamental electromagnetic principles. This combined approach enables the development of a model that can generalize effectively to unseen scenarios while maintaining physical accuracy.

The trained neural operator model resulting from the model training process 40 may be implemented as the neural network module 14 in the system architecture 10 described in relation to FIG. 1a and FIG. 1b.

FIG. 5 illustrates a method for electromagnetic field simulation 50 using a non-autoregressive neural operator model. The method 50 comprises a sequence of steps for processing input data and generating a simulated electromagnetic field output.

The method 50 begins with an input step 51, which involves inputting device geometry and parameters (e.g. input data representing the medical device geometry of the medical device, at least one operating parameter of the medical device, and an operating environment of the medical device). The data represents the three-dimensional configuration of an ablation device and its operating conditions. As defined earlier, these operating conditions may comprise two parts: the geometry of the area of the patient where the device is to be applied (the "3D material parameters" including 3D image data and 3D tissue properties such as electrical conductivity, thermal conductivity, and perfusion rates) and the variable parameters used to operate the medical device (including power level, frequency, duration of application, pulse width, duty cycle, cooling flow rate, electrode configuration, and treatment mode selection). This input data may be provided through the geometry input 11, parameter input 12, and environment input 13 of the system architecture 10 described in relation to FIG. 1a and FIG. 1b.

In a input processing step 52, the method 50 processes the inputs through a branch network. The step 52 transforms the multidimensional input data through the branch network 212 portion of the neural operator model, as described in relation to FIG. 2. The branch network 212 may include components such as the initial condition input 21, boundary condition input 22, material parameters input 23, and probe geometry input 24, which feed into the neural network processor 25 and branch embedding module 26.

The method 50 then proceeds to a query points generation step 53, where query points are generated for desired simulation locations and times. The step 53 defines the spatial and temporal coordinates at which the electromagnetic field will be evaluated. These query points may correspond to the coordinate input module 27 of the trunk network 213 described in FIG. 2.

In a query points processing step 54, the method 50 processes the query points through a trunk network. The step 54 transforms the spatial and temporal coordinates through the trunk network 213 portion of the neural operator model. This processing may involve the perceptron processor 28 and trunk embedding module 29 as described in relation to FIG. 2.

The method 50 continues to attention step 55, where a dot product calculation is performed to produce electromagnetic field values (more generally speaking, an attention mechanism is applied to combine the branch and trunk outputs). The step 55 combines the processed information from both the branch network 212 and trunk network 213 to predict field values at the specified coordinates. This step corresponds to the attention mechanism operation 210 described in FIG. 2.

Finally, in output step 56, the method 50 outputs the simulated electromagnetic field. The step 56 produces a representation of the electromagnetic field distribution generated by the medical device under the specified conditions. This output may correspond to the field output 211 described in FIG. 2 and may be equivalent to the simulated EM field 15 in the system architecture 10 of FIG. 1a and FIG. 1b.

The simulated electromagnetic field output from step 56 may be used by the calculation module 16 to derive device functional parameters, such as the dimensions of the ablation zone 30 described in relation to FIG. 3. These parameters may include the ablation length 34, ablation long width 35, ablation short width 36, and distal boundary 37 of the ablation zone 30 generated by the ablation device 31.

The method 50 may be implemented as part of the neural network module 14 in the system architecture 10. The output from the method 50 may be used in various applications, such as device design optimization through the optimization subsystem 18, where the simulated electromagnetic field may be compared to a desired electromagnetic field pattern 181 by the loss calculator 182, and the optimizer 183 may use this information to adjust input parameters through the feedback path 184.

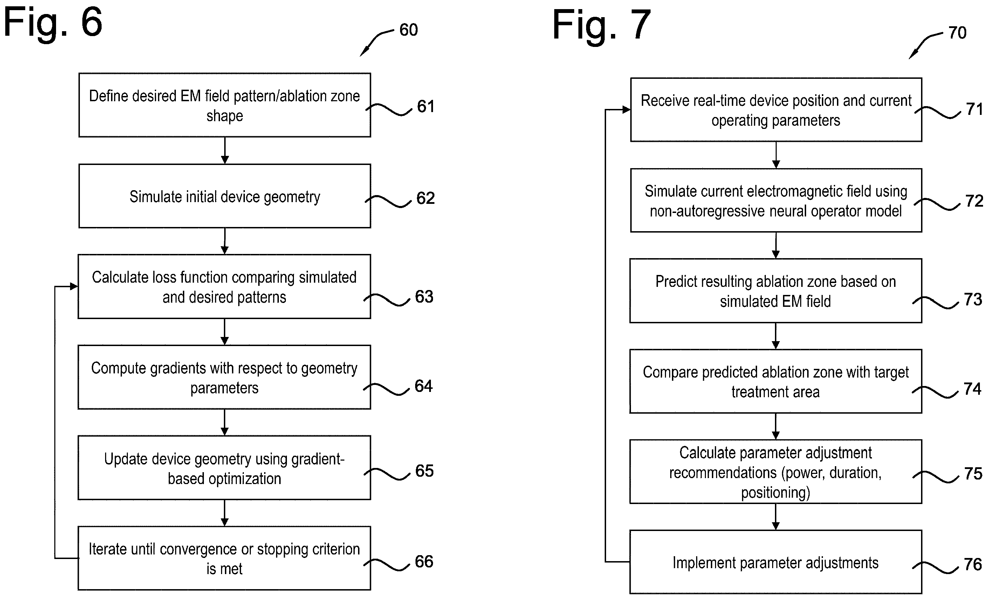

FIG. 6 illustrates a device design optimization method 60 for refining the geometry of a medical device, such as an ablation device 31, through iterative electromagnetic field simulation and gradient-based optimization.

A field shape definition step 61 of the method 60 involves defining a desired electromagnetic field pattern. The desired electromagnetic field pattern may correspond to specific therapeutic objectives, such as achieving a spherical ablation zone 30 instead of the typical ellipsoidal shape. The desired field pattern may be specified using the desired electromagnetic field pattern 181 component of the optimization subsystem 18 described in relation to the system architecture 10.

An initial geometry simulation step 62 of the method 60 involves simulating an initial device geometry by providing initial input data representing a medical device geometry and using the trained non-autoregressive neural operator model. The initial input data representing the medical device geometry may be provided through the geometry input 11 of the system architecture 10. The neural network module 14 processes the initial geometry along with operational parameters from the parameter input 12 and environmental conditions from the environment input 13 to generate a simulated EM field 15.

A loss function calculation step 63 of the method 60 involves calculating a loss function based on the difference between the simulated electromagnetic field and the desired electromagnetic field pattern. The loss function quantifies the discrepancy between the current field distribution and the target distribution. This calculation may be performed by the loss calculator 182 of the optimization subsystem 18.

A gradients computation step 64 of the method 60 involves computing gradients with respect to medical device geometry parameters. The gradients indicate how small changes in the device geometry affect the electromagnetic field distribution. The computation of gradients leverages the differentiability of the neural network module 14, allowing for efficient backpropagation of error gradients through the simulation pipeline.

A device geometry update step 65 of the method 60 involves updating the input data representing the medical device geometry using gradient-based optimization techniques to minimize the calculated loss function. The optimizer 183 of the optimization subsystem 18 may perform this step, determining parameter adjustments that reduce the discrepancy between the simulated and desired field patterns.

An iteration step 66 of the method 60 involves iterating the process until convergence or until a stopping criterion is met. The method 60 includes a feedback loop from step 66 back to step 63, indicating that the loss function calculation, gradient computation, and geometry updates continue iteratively. The feedback loop may be implemented through the feedback path 184 of the optimization subsystem 18, which communicates parameter adjustments back to the input components of the system architecture 10.

The device design optimization method 60 may be applied to various aspects of the ablation device 31 geometry, including the dimensions and shape of the antenna shaft 32 and antenna tip 33. The method 60 may aim to optimize parameters such as the ablation length 34, ablation long width 35, ablation short width 36, and distal boundary 37 of the resulting ablation zone 30.

In an example, the device design optimization method 60 includes manufacturing a physical ablation device based on the optimized design specifications produced by the iterative optimization process. The optimized parameters determined by the method 60 may be applied to control computer-aided manufacturing equipment for producing the physical device. Alternatively, for existing device platforms, the optimization results may be used to adjust configurable parameters or to create modified components that can be incorporated into the device assembly.

The device design optimization method 60 leverages the computational efficiency and differentiability of the non-autoregressive neural operator model implemented in the simulation system 20. By enabling rapid iterations of electromagnetic field simulation and gradient-based optimization, the method 60 facilitates the exploration of complex device geometries and operational parameters that may be challenging to evaluate using traditional electromagnetic simulation approaches.

FIG. 7 illustrates a parameter adjustment method 70 for real-time treatment parameter adjustment during an ablation procedure. The method 70 comprises a sequence of steps designed to provide continuous monitoring and adjustment of treatment parameters based on updated electromagnetic field simulations.

A real-time data reception step 71 of the method 70 involves receiving real-time device position and current operating parameters (e.g. input data representing at least one operating parameter of the medical device, and an operating environment of the medical device). Input data representing the medical device geometry may be provided once (as it will typically not change during the procedure) or regularly (e.g. at each iteration). Likewise, operating environment data such as medical images, tissue characteristics, and tumor location information obtained from the patient data module 912 of the system architecture 90 may be provided once or regularly. The step 71 may receive data from sensors or imaging systems that track the spatial coordinates of the ablation device 31 within the patient's body. The step 71 may also receive updated operational settings from the parameter input 12 of the system architecture 10.

A current field simulation step 72 of the method 70 involves simulating the current electromagnetic field using the non-autoregressive neural operator model. The step 72 utilizes the neural network module 14 to rapidly generate a simulated EM field 15 based on the updated input data received in step 71. The simulation process may involve the branch network 212 processing updated geometry and material parameters, while the trunk network 213 handles the new spatial coordinates.

An ablation zone prediction step 73 of the method 70 involves predicting the resulting ablation zone based on the simulated electromagnetic field. The step 73 may use the calculation module 16 to process the simulated EM field 15 and estimate the dimensions of the ablation zone 30, including the ablation length 34, ablation long width 35, and ablation short width 36.

A comparison step 74 of the method 70 involves comparing the predicted ablation zone with the target treatment area. The step 74 may evaluate how closely the current ablation parameters align with the intended treatment objectives, such as coverage of a tumor volume or avoidance of critical structures.

A parameter adjustment calculation step 75 of the method 70 involves calculating parameter adjustment recommendations. The step 75 may use the optimization subsystem 18 to determine modifications to power settings, ablation duration, or device positioning that may improve the alignment between the predicted and desired ablation zones.

A parameter adjustment implementation step 76 of the method 70 involves implementing the calculated parameter adjustments to adjust the treatment parameters. The step 76 may communicate the recommended changes to the ablation device 31 as parameter updates or provide visual or audio guidance to the clinician for manual adjustment.

The method 70 includes a feedback loop from step 76 back to step 71, creating a continuous cycle of monitoring and adjustment throughout the procedure. This iterative process allows for real-time adaptation of the treatment strategy based on the most current data and simulations. The method 70 may be performed in real-time during a medical procedure to provide treatment guidance. The rapid simulation capabilities of the neural network module 14 enable the method 70 to process updated input data, re-simulate the electromagnetic field, and provide real-time feedback for adjusting treatment parameters with minimal latency.

By continuously updating the electromagnetic field simulation and ablation zone prediction based on the current device position and parameters, the method 70 allows clinicians to make informed decisions and adjustments throughout the course of the ablation procedure. This real-time guidance may help ensure that the actual ablation zone 30 closely matches the intended treatment area, potentially improving the efficacy and safety of the procedure.

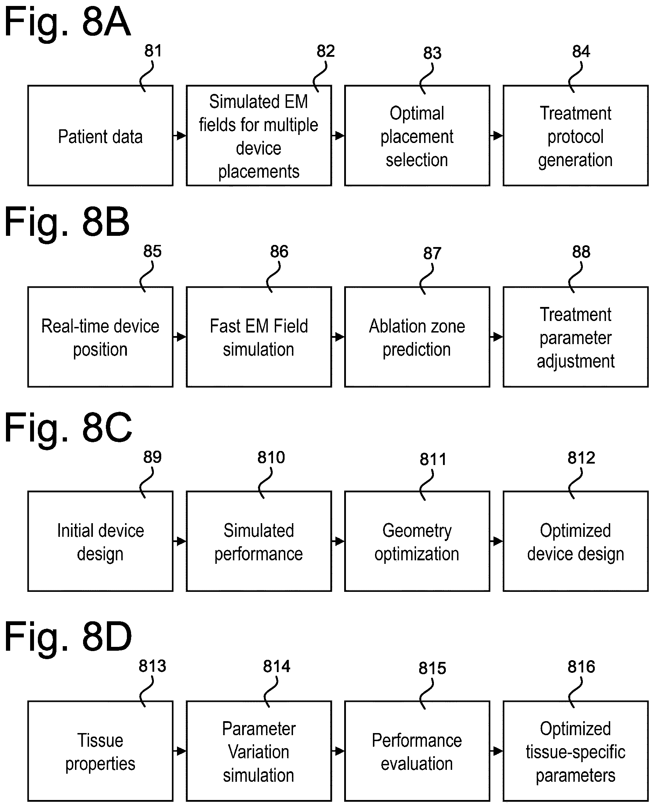

FIG. 8a illustrates a pre-procedure planning workflow for optimizing ablation treatment parameters prior to a medical procedure. The workflow comprises a sequence of processing steps designed to generate a patient-specific treatment protocol based on electromagnetic field simulations.

The workflow begins with a patient information step 81 configured to receive and process patient-specific information. The step 81 may include data such as medical images, tissue characteristics, and tumor location information obtained from the patient data module 912 of the system architecture 90. The step 81 may process this information to create a digital representation of the patient's anatomy and target treatment area. This data can serve as input data representing the operating environment of the medical device. In addition, input data representing the medical device geometry and at least one initial operating parameter of the medical device may be provided.

The output from the step 81 flows to an EM field generation step 82, which generates electromagnetic field simulations for multiple potential device placements. The step 82 may utilize the neural network module 14 to rapidly simulate electromagnetic fields for various configurations of the ablation device 31 within the patient's anatomy. The step 82 may leverage the simulation system 20, including the branch network 212 and trunk network 213, to efficiently process different geometric configurations and generate corresponding simulated EM field 15.

The simulated electromagnetic field data from the step 82 is provided to a placement selection step 83. The step 83 analyzes the simulated fields to determine optimal device positioning. This analysis may involve evaluating factors such as the ablation zone 30 coverage, proximity to critical structures, and minimization of damage to healthy tissue. The step 83 may utilize the calculation module 16 to derive relevant metrics from the simulated electromagnetic fields, such as the ablation length 34, ablation long width 35, and ablation short width 36 for each potential placement.

Following the placement selection, the workflow proceeds to a treatment protocol generation step 84. The step 84 formulates treatment parameters based on the selected optimal placement. These parameters may include specific settings for the parameter input 12, such as power levels, frequency, and duration of the ablation procedure. The step 84 may also define the precise positioning coordinates for the ablation device 31 based on the analysis performed in the step 83.

The workflow represents a sequential process where data flows from the step 81 through the step 82 and step 83 to ultimately generate a treatment protocol in the step 84. This pre-procedure planning workflow enables clinicians to evaluate multiple potential treatment strategies virtually before performing the actual procedure, potentially improving treatment outcomes and reducing procedural risks.

By simulating multiple electromagnetic fields for different medical device placements, evaluating these simulated fields, and generating a comprehensive treatment protocol, the workflow implements a data-driven approach to treatment planning. This method, performed as part of a pre-procedure treatment planning process, leverages the computational efficiency of the neural network module 14 to explore a wide range of treatment options and identify the most promising approach for each individual patient.

FIG. 8b illustrates an intra-procedure guidance workflow for real-time electromagnetic field simulation and treatment parameter adjustment during an ablation procedure. The workflow comprises a sequence of processing steps that enable continuous monitoring and adaptation of treatment parameters based on the current position and settings of the ablation device 31.

The workflow begins with a real-time device positioning step 85, which represents real-time device position processing involving receiving and processing positional data of the ablation device 31 during the procedure. The positional data may be obtained from imaging systems, position sensors, or tracking devices that monitor the location and orientation of the ablation device 31 within the patient's body. The positional data may be part of the input data representing the operating environment of the medical device, in particular the medical device positioning and orientation with respect to the patient to be treated. The step 85 may also process updated operational settings (input representing at least one operating parameter of the medical device) from the parameter input 12 of the system architecture 10.

Following the device position processing, the workflow proceeds to an EM field simulation step 86, which represents fast electromagnetic field simulation. The step 86 utilizes the neural network module 14 to rapidly simulate the electromagnetic field generated by the ablation device 31 at the current position and with the current operational settings. The step 86 may employ the branch network 212 to process the updated geometry and material parameters, while the trunk network 213 handles the new spatial coordinates. The simulation process in step 86 generates a simulated EM field 15 that represents the current electromagnetic field distribution surrounding the ablation device 31.

The workflow then advances to an ablation zone prediction step 87, which represents ablation zone prediction. The step 87 processes the simulated EM field 15 to predict the expected ablation zone 30 that would result from the current device position and settings. The step 87 may utilize the calculation module 16 to derive the dimensions of the predicted ablation zone 30, including the ablation length 34, ablation long width 35, and ablation short width 36. The ablation zone prediction may incorporate biophysical models for heat propagation and cell necrosis to provide a comprehensive estimate of the tissue affected by the ablation procedure.

The final step in the workflow is a treatment parameter adjustment step 88, which represents treatment parameter adjustment. The step 88 involves analyzing the predicted ablation zone 30 and determining appropriate adjustments to treatment parameters based on the analysis. The step 88 may compare the predicted ablation zone 30 with the target treatment area, evaluating factors such as coverage of the tumor volume and proximity to critical structures. Based on this comparison, the step 88 may calculate recommended modifications to power settings, ablation duration, or device positioning to optimize the treatment outcome. The step 88 may utilize the optimization subsystem 18 to determine these parameter adjustments, potentially employing the optimizer 183 to minimize the discrepancy between the predicted and desired ablation zones.

The intra-procedure guidance workflow enables real-time adaptation of the ablation procedure based on the most current data and simulations. The rapid simulation capabilities of the neural network module 14 allow for near-instantaneous processing of updated input data, re-simulation of the electromagnetic field, and generation of parameter adjustment recommendations. This continuous feedback loop may assist clinicians in making informed decisions throughout the course of the ablation procedure, potentially improving the precision and efficacy of the treatment.

By providing real-time visualization and quantitative predictions of the ablation zone 30, the workflow may enhance the clinician's ability to ensure complete coverage of the target tissue while minimizing damage to surrounding healthy structures. The method may include implementing the calculated parameter adjustments to adjust the treatment parameters, either automatically or through a visual or audible signal to the clinician.

FIG. 8c illustrates a device design optimization workflow for refining the geometry and operational parameters of an ablation device. The workflow comprises a sequence of steps for iteratively improving device performance through electromagnetic field simulation and optimization techniques.

The workflow begins with an initial device design step 89 configured to receive and process data defining a preliminary geometric configuration of an ablation device. The step 89 may utilize the geometry input 11 of the system architecture 10 to input initial device specifications, such as dimensions and shape of the antenna shaft 32 and antenna tip 33. These specifications serve as the initial input data representing the medical device geometry. In addition, input data representing at least one operating parameter of the medical device, and an operating environment of the medical device may be provided.

The output from the step 89 flows to a performance simulation step 810, which utilizes the neural network module 14 to simulate the electromagnetic field generated by the initial device design and evaluate performance characteristics. The step 810 may employ the branch network 212 to process the device geometry and material parameters, while the trunk network 213 handles spatial coordinates for field evaluation. The step 810 generates a simulated EM field 15 representing the electromagnetic field distribution produced by the initial device configuration.

The simulated electromagnetic field data from the step 810 is transmitted to a step 811, which applies gradient-based optimization techniques to the device design parameters. A step 811 may utilize the optimization subsystem 18 to analyze the simulated field and compare the performance to desired outcomes. The step 811 may employ the loss calculator 182 to quantify discrepancies between the simulated field and target field patterns defined by the desired electromagnetic field pattern 181. The optimizer 183 may then calculate gradients with respect to design parameters and determine modifications to minimize the computed loss function.

A geometry optimization step 811 iteratively modifies the input representing the medical device geometry and the at least one operational parameter to improve performance. The step 811 may adjust parameters such as the dimensions of the antenna shaft 32 and antenna tip 33, aiming to achieve desired characteristics of the ablation zone 30, such as the ablation length 34, ablation long width 35, and ablation short width 36.

Upon convergence to an optimal solution or reaching a predefined number of iterations, the step 811 communicates the refined design specifications to a design finalization step 812. The step 812 finalizes the optimized device configuration, incorporating any manufacturing constraints or clinical requirements. The step 812 may produce a comprehensive set of specifications for the optimized ablation device, including detailed geometric parameters and recommended operational settings.

The workflow represents a sequential process where data flows from the step 89 through the steps 810 and 811 to ultimately generate an optimized device design in the step 812. The feedback path 184 of the optimization subsystem 18 may facilitate iterative refinement by communicating updated parameters back to earlier stages of the workflow for subsequent simulation and optimization cycles.

By leveraging the computational efficiency of the neural network module 14 and the gradient-based optimization capabilities of the optimization subsystem 18, the device design optimization workflow enables rapid exploration of complex device geometries and operational parameters. The workflow may facilitate the development of ablation devices with improved performance characteristics, such as more precise control over the ablation zone 30 or enhanced energy delivery efficiency.

The method may include manufacturing the optimized medical device design. In some aspects, the device design optimization workflow may include an additional step that generates device manufacturing parameters based on the optimized device design. This step may translate the geometric specifications and material requirements of the optimized ablation device into a format suitable for direct input into manufacturing equipment.

The workflow may interface with computer-aided manufacturing (CAM) software to produce machine-specific instructions for fabricating the optimized device. For example, the system may generate G-code or other numerical control programming languages compatible with various manufacturing technologies such as 3D printers, CNC machines, or injection molding equipment.

In some implementations, the manufacturing parameter generation step may take into account specific constraints and capabilities of the target manufacturing process. This may include considerations such as minimum feature sizes, material properties, and build orientations for additive manufacturing processes like 3D printing.

The system may also output specifications for different components of the ablation device that may require distinct manufacturing processes. For instance, the antenna shaft 32 and antenna tip 33 may have different material requirements or geometric complexities that necessitate separate manufacturing steps or techniques.

By extending the optimization workflow to include manufacturing parameter generation, the system may provide a more comprehensive end-to-end solution that bridges the gap between device design and physical production. This integration may potentially streamline the product development cycle, reduce manufacturing errors, and ensure that the optimized design characteristics are accurately translated into the final manufactured device.

The workflow may also comprise the actual manufacture of the device. In some implementations, the system may interface directly with manufacturing equipment to initiate and control the production process. This integration may allow for seamless transition from design optimization to physical fabrication.

The manufacturing step may involve various processes depending on the specific device design and materials. For example, the system may coordinate 3D printing of complex geometric features, CNC machining of precision components, or injection molding of high-volume parts. In some cases, the workflow may orchestrate a combination of manufacturing techniques to produce different elements of the ablation device.

During the manufacturing process, the system may continuously monitor production parameters and compare them to the optimized design specifications. This real-time monitoring may enable adaptive adjustments to manufacturing settings, potentially improving production quality and consistency. The system may also incorporate in-line quality control measures, using sensors or imaging systems to verify that manufactured components meet the required tolerances and performance characteristics.

In some aspects, the manufacturing step may include assembly processes for multicomponent devices. The workflow may generate assembly instructions and potentially interface with robotic systems to automate precise assembly operations. This may help ensure that the final assembled device accurately reflects the optimized design parameters.

By incorporating the actual manufacturing process into the workflow, the system may provide a closed-loop optimization and production pipeline. This integration may allow for rapid prototyping and iterative refinement of device designs based on real-world manufacturing outcomes, potentially accelerating the development cycle for new ablation devices.

FIG. 8d illustrates a tissue-specific parameter optimization workflow for refining ablation device parameters based on specific tissue characteristics. The workflow comprises a sequence of steps designed to determine optimal operational settings for different tissue types.

The workflow begins with a tissue property input step 813 configured to receive and process data characterizing the properties of specific tissue types. This data may serve as the input data representing an operating environment of the medical device. The step 813 may utilize the patient data module 912 of the system architecture 90 to input tissue-specific information such as electrical conductivity, thermal conductivity, and perfusion rates. The step 813 may process this information to create a digital representation of the tissue environment for electromagnetic field simulation.

The output from the step 813 flows to a parameter variation simulation step 814, which performs parameter variation simulations using different combinations of operational parameters (also referred to as a set of at least one operating parameter of the medical device). The step 814 may employ the neural network module 14 to rapidly simulate electromagnetic fields for various configurations of the ablation device 31 within the specified tissue environment. The step 814 may leverage the simulation system 20, including the branch network 212 and trunk network 213, to efficiently process different parameter sets and generate corresponding simulated EM field 15.

The simulated electromagnetic field data from the step 814 is provided to a performance evaluation step 815. The step 815 analyzes the effectiveness of different parameter configurations within the context of the specific tissue environment. The step 815 may utilize the calculation module 16 to derive relevant metrics from the simulated electromagnetic fields, such as the ablation length 34, ablation long width 35, and ablation short width 36 for each parameter set. The step 815 may evaluate factors such as ablation zone 30 size, shape, uniformity, and margin adequacy relative to target tissue volumes.

Following the performance evaluation, the workflow proceeds to a determination step 816. A step 816 determines the optimal set of the at least one operating parameter of the medical device for the particular tissue type under consideration. The step 816 may compile the optimal set, which may include power levels, frequency modulation, ablation duration, and cooling protocols specifically tailored to achieve optimal therapeutic outcomes in the target tissue type.

The workflow enables the optimization of device parameters for specific tissue types, such as fatty or cirrhotic liver tissue. By simulating the electromagnetic field for multiple sets of device parameters and evaluating the resulting field distributions, the workflow can identify sets of at least one operating parameter of the medical device that maximize treatment efficacy while accounting for the unique properties of different tissue environments.

The optimized tissue-specific parameters determined by the step 816 may be stored in the model repository 911 or device design module 913 of the system architecture 90. This storage allows for subsequent retrieval during clinical procedures involving the corresponding tissue type, enabling personalized treatment approaches that account for tissue-specific variability in electromagnetic and thermal response characteristics.

The tissue-specific parameter optimization workflow represents a data-driven approach to tailoring ablation treatments to individual patient characteristics. By leveraging the computational efficiency of the neural network module 14 and the analytical capabilities of the calculation module 16, the workflow facilitates the development of optimized treatment protocols for diverse tissue environments, potentially enhancing the precision and efficacy of ablation procedures across varying anatomical locations and pathological conditions.

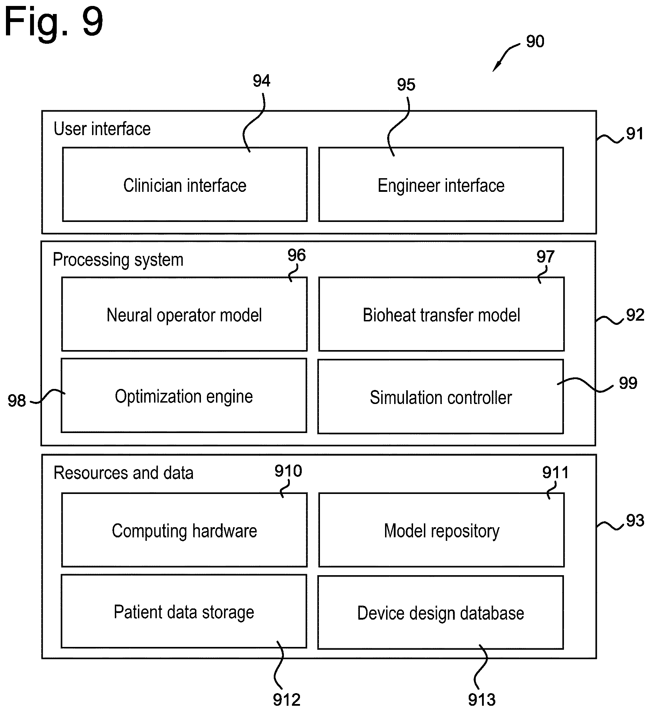

FIG. 9 illustrates a system architecture 90 for electromagnetic field simulation comprising three layers: an interface layer 91, a processing layer 92, and a resource layer 93.

The interface layer 91 includes a clinician interface module 94 and an engineer interface module 95. The clinician interface module 94 provides tools for treatment planning and procedure guidance, while the engineer interface module 95 facilitates device design and parameter configuration. These separate interfaces allow for tailored interactions based on the specific needs of clinicians and engineers.

The processing layer 92 contains a neural operator module 96 for electromagnetic field simulations, a bioheat transfer module 97 for temperature calculations, an optimization module 98 for parameter optimization, and a simulation controller module 99 for coordinating execution between components. The neural operator module 96 may implement the neural network module 14 described in previous figures, while the bioheat transfer module 97 may incorporate the calculation module 16 for deriving thermal effects from the simulated electromagnetic fields. The optimization module 98 may correspond to the optimization subsystem 18, including components such as loss calculator 182 and optimizer 183.

The resource layer 93 comprises a computing hardware module 910 for processing operations, a model repository module 911 for storing trained models, the patient data module 912 for maintaining patient information, and the device design module 913 for storing device specifications and parameters. The computing hardware module 910 may include central processing units (CPUs) for general-purpose computation and graphics processing units (GPUs) for accelerated parallel computation of neural network operations. The use of GPUs enables efficient processing of the complex calculations required by the neural operator module 96 and other components of the processing layer 92.

The layers are arranged hierarchically with bidirectional data flow between adjacent layers, allowing user inputs to propagate through the processing layer 92 to the resource layer 93, and computational results to be transmitted back through the processing layer 92 for presentation at the interface layer 91. For example, a clinician may input patient data and treatment parameters through the clinician interface module 94, which are then processed by the neural operator module 96 and bioheat transfer module 97 to generate a simulated EM field 15 and predicted ablation zone 30. The results may then be displayed back to the clinician through the clinician interface module 94 for treatment planning or intra-procedural guidance.

The system architecture 90 supports various workflows described in previous figures. For instance, the pre-procedure planning workflow of FIG. 8a may utilize the patient data module 912 to provide input for step 81, while the neural operator module 96 and simulation controller module 99 may facilitate the electromagnetic field simulations in step 82. The intra-procedure guidance workflow of FIG. 8b may leverage the real-time processing capabilities of the computing hardware module 910 to execute steps 85-88 with minimal latency.

The modular design of the system architecture 90 allows for flexible deployment across various computing environments while maintaining consistent functionality. The separation of concerns between the interface, processing, and resource layers enables independent scaling and optimization of each layer as needed.

A computer device implementing the system architecture 90 comprises a processor and a memory, where the memory comprises instructions stored thereon. When executed by the processor, these instructions cause the computer device to perform the methods described in previous figures, such as the electromagnetic field simulation method 50 of FIG. 5 or the device design optimization method 60 of FIG. 6.

The system architecture 90 may be implemented as a computer program comprising instructions which, when executed by a computer, cause the computer to carry out the various methods and processes described herein. The computer program may be stored on a computer-readable medium, such as the model repository module 911 or other storage components within the resource layer 93.

By providing a comprehensive framework for electromagnetic field simulation, thermal modeling, and device optimization, the system architecture 90 enables efficient development and refinement of ablation devices 31 and treatment protocols. The architecture's flexibility and computational power support a wide range of applications, from pre-procedure planning to real-time treatment guidance and post-procedure analysis.

The systems and methods described herein may be implemented in any form of computing or electronic device. The term "computer," as used herein, encompasses any device with processing capabilities sufficient to execute instructions. This includes, but is not limited to, personal computers, servers, mobile devices, personal digital assistants, and similar devices.

Such devices may include one or more processors, such as microprocessors, controllers, or other suitable types of processors, capable of executing instructions to control the device's operation. For example, in some implementations using a system-on-a-chip architecture, the processors may include fixed-function blocks (hardware accelerators) that perform parts of the method in hardware rather than software or firmware. Platform software, such as an operating system or similar, may be installed to support the execution of application software.

The described functionality may be implemented in hardware, software, or any combination thereof. When implemented in software, the instructions or code can be stored on or transmitted via a computer-readable medium. Such media include computer-readable storage media, which may be volatile or non-volatile, removable or non-removable, and implemented using any technology for storing information such as program code, data structures, or other data. Examples include, but are not limited to, ROM, EEPROM, RAM, magnetic or optical storage, flash memory, or any other storage medium accessible by a computer. Communication media that facilitate the transfer of software, such as via coaxial cables, fiber optics, DSL, or wireless signals, may also be considered part of computer-readable media.

Alternatively, or in addition, some or all of the described functionality may be implemented using hardware logic components. Examples include, but are not limited to, application-specific integrated circuits, system-on-a-chip systems, field-programmable gate arrays, application-specific standard products, and complex programmable logic devices. In some cases, software instructions may also be implemented in dedicated circuits, such as programmable logic arrays or digital signal processors.

The computing device may operate as a standalone system or as part of a distributed system, where tasks are performed collectively by multiple devices connected via a network. Such devices may communicate over a network connection to perform the described functionality. For instance, software may be stored on a remote computer and accessed by a local device, which may download and execute portions of the software as needed. Similarly, some instructions may be processed locally, while others may execute on remote systems or networks. In some cases, the computing device may be remote and accessible via a communication interface. Storage of program instructions may also be distributed across a network or stored in a combination of local and remote locations. For example, software may reside on a remote computer and be accessed by a local terminal, or the system may execute some software locally while other components operate on remote servers.

Features of any of the examples or embodiments outlined above may be combined to create additional examples or embodiments without losing the intended effect. It should be understood that the description of an embodiment or example provided above is by way of example only, and various modifications could be made by one skilled in the art. Furthermore, one skilled in the art will recognize that numerous further modifications and combinations of various aspects are possible. Accordingly, the described aspects are intended to encompass all such alterations, modifications, and variations that fall within the scope of the appended claims.

## Claims

1. A computer-implemented method for electromagnetic field simulation, comprising:
receiving input data representing a medical device geometry of a medical device, at least one operating parameter of the medical device, and an operating environment of the medical device;
processing the input data using a trained neural operator model to determine a simulated electromagnetic field (15) as generated by the medical device with the at least one operating parameter and in the operating environment; and
calculating, based on the simulated electromagnetic field (15), at least one functional parameter of the medical device.

2. The method of claim 1, wherein the medical device is an ablation device (31), and wherein the at least one functional parameter comprises a parameter of an ablation zone (30) of the ablation device and/or a power density within the ablation zone (30) and/or a parameter of the simulated electromagnetic field (15).

3. The method of claim 1 or 2, wherein the trained neural operator model is a non-autoregressive neural operator model and comprises:
a branch network (212) configured to process the input data;
a trunk network (213) configured to process space and time sampling locations; and
an attention mechanism (210) combining the branch network (212) and trunk network (213).

4. The method of any of claims 1 to 3, wherein the branch network (212) is configured to process input data in at least one format selected from a 3D grid, a finite element mesh, and a point cloud.

5. The method of any of claims 1 to 4, wherein the trained neural operator model has been trained using a combination of supervised learning with reference electromagnetic field data and physics-informed learning based on Maxwell's equations.

6. The method of any of claims 1 to 5, further comprising:
optimizing the medical device geometry by:
defining a desired electromagnetic field pattern (181);
calculating a loss function based on a difference between the simulated electromagnetic field (15) and the desired electromagnetic field pattern (181); and
updating the input data representing the medical device geometry using a gradient-based optimization method to minimize the loss function.

7. The method of claim 6, wherein the medical device is an ablation device (31) and the desired electromagnetic field pattern (181) corresponds to a spherical ablation zone (30) of the ablation device.

8. The method of any of claims 1 to 5, wherein the method is performed in real-time during a medical procedure to provide treatment guidance, the method further comprising:
receiving updated input data reflecting at least one of an updated operating parameter of the medical device and an updated operating environment of the medical device;
re-simulating the electromagnetic field based on the updated input data; and
providing real-time feedback for adjusting treatment parameters.

9. The method of any of claims 1 to 5, wherein the method is performed as part of a pre-procedure treatment planning process, the method further comprising:
simulating multiple electromagnetic fields for different medical device placements.
evaluating the simulated electromagnetic fields to determine an optimal medical device placement; and
generating a treatment protocol based on the optimal medical device placement.

10. The method of any of claims 1 to 5, further comprising:
optimizing device parameters for a specific tissue type by:
simulating the electromagnetic field for multiple sets of at least one operating parameter of the medical device;
evaluating the simulated electromagnetic field to determine an optimal set of the at least one operating parameter of the medical device for the specific tissue type; and
storing the optimal set of the at least one operating parameter of the medical device for future use with the specific tissue type.

11. The method of any of claims 1 to 10, further comprising:
integrating the simulated electromagnetic field (15) with a biophysical model (97) for heat propagation and cell necrosis to predict a resulting ablation zone (30).

12. The method of any of claims 1 to 11, further comprising:
optimizing the at least one operating parameter of the medical device to maximize overlap between a predicted ablation zone (30) and a target tumor volume.

13. A computer device comprising a processor and a memory, wherein the memory comprises instructions stored thereon which, when executed by the processor, cause the computer device to perform the method of any of claims 1 to 12.

14. A computer program comprising instructions which, when executed by a computer, cause the computer to carry out the method of any of claims 1 to 12.

15. A computer-readable medium comprising instructions which, when executed by a computer, cause the computer to carry out the method of any of claims 1 to 12.
